(19) Europäisches Patentamt
European Patent Office
Office européen des brevets

(11) Publication number: **0 274 876**
**B1**

(12) **EUROPEAN PATENT SPECIFICATION**

(45) Date of publication of patent specification: **30.01.91**

(51) Int. Cl.$^5$: **C 07 C 329/16**

(21) Application number: **87311015.9**

(22) Date of filing: **15.12.87**

(54) **Process for preparing bis(ethoxythiocarbonyl)sulfide.**

(30) Priority: **18.12.86 US 943536**

(43) Date of publication of application:
**20.07.88 Bulletin 88/29**

(45) Publication of the grant of the patent:
**30.01.91 Bulletin 91/05**

(84) Designated Contracting States:
**AT BE CH DE ES FR GB GR IT LI LU NL SE**

(56) References cited:
**US-A-4 354 980**

**ANALES DE QUIMICA, series C, vol. 76, 1980, pages 183-186, Madrid, ES; M. HERRERA et al.: "La reaccion del O-etilditiocarbonato de S-etoxicarbonilo con aminas alifaticas"**

(73) Proprietor: **PFIZER INC.**
**235 East 42nd Street**
**New York, N.Y. 10017 (US)**

(72) Inventor: **Sklavounos, Constantine**
**7, East Wharf Road**
**Waterford Connecticut (US)**

(74) Representative: **Bradbrook, Geoffrey William et al**
**PFIZER LIMITED Ramsgate Road**
**Sandwich Kent, CT13 9NJ (GB)**

**EP 0 274 876 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Bis(ethoxythiocarbonyl)sulfide, of the formula

$$C_2H_5O\overset{\overset{S}{\|}}{C}-S-\overset{\overset{S}{\|}}{C}-OC_2H_5$$

has been employed as an intermediate in the synthesis of ethoxythiocarbonyl derivatives of amino acids and their esters, which, in turn, are useful in peptide synthesis. Of the various literature procedures leading to bis(ethoxythiocarbonyl)sulfide, that reported by Barany, et al. provides the desired product in about 57% yield (*J. Orga. Chem., 43,* 2930, 1978).

It has now been found that bis(ethoxythiocarbonyl)sulfide can be prepared in nearly quantitative yields by a process which comprises contacting an aqueous-ethanolic solution of sodium ethyl xanthate with ethyl chloroformate in a molar ratio of 2.1—2.3 moles of sodium ethyl xanthate to 1 mole of ethyl chloroformate and maintaining the pH of the resulting reaction mixture at about 7.5 by the addition of acid until the reaction is substantially complete.

It is preferred in the instant process that the volume ratio of ethanol to water be from about 2—3 to about 3—2.

It is further preferred that the pH be maintained by the addition of concentrated hydrochloric acid.

The starting reagents for the present process, sodium ethyl xanthate and ethyl chloroformate, are combined in a reaction solvent consisting of ethanol and water. Sufficient water is necessary to solubilize the sodium ethyl xanthate and sufficient ethanol is required to solubilize the ethyl chloroformate. It is preferred that a volume ratio of ethanol to water of from about 2—3 to about 3—2 will meet the aforementioned requirements and allow the reaction to proceed to completion in a practical reaction time.

The sodium ethyl xanthate can be added to an aqueous ethanolic solution followed by the addition of the ethyl chloroformate or, for convenience sake, the sodium ethyl xanthate can be prepared from carbon disulfide and sodium hydroxide in an ethanol solution and water added at the completion of the reaction to provide the aqueous-ethanolic reaction solution to which the ethyl chloroformate is added.

The sodium ethyl xanthate and ethyl chloroformate are combined in a molar ratio of 2.1—2.3 moles of xanthate per mole of formate. On completion of the combining of the reactants, the pH is monitored and 'maintained at' about 7.5 by the addition of acid. Any acid capable of adjusting the pH to 7.5 is suitable, although strong acids such as organic sulfonic acids or mineral acids are preferred.

The maintenance of the pH at 7.5 leads to the decomposition of a reaction by-product, ethyl thiocarbonate. By removing this by-product a higher yield of the desired product is obtained. In addition, the product which is obtained, being free of unwanted contaminants, does not require further purification before use.

At ambient temperatures the reaction is complete in about 6 hours and there is little change in the pH of the reaction mixture. The product, which forms as yellow crystals, can be filtered directly from the reaction.

The following examples are provided solely for the purpose of further illustration.

### Example 1

Bis(ethoxythiocarbonyl)sulfide

To a thick white slurry resulting from 20.82 g (.51 mole) of sodium hydroxide pellets and 74 ml of 95% ethanol was added 40.36 g (.53 mole) of carbon disulfide dropwise over a period of 15 minutes. The temperature of the reaction mixture was maintained at about 22C by cooling with an ice bath.

The resulting thick, orange slurry (sodium ethyl xanthate) was dissolved with the addition of 111 ml of water to produce a clear orange solution. Ethyl chloroform (24.06 g, 0.222 mole) was then added over a period of about 2 minutes. The mixture was stirred at ambient temperatures while concentrated hydrochloric acid was gradually added (total 17.8 ml) to maintain the pH at 7.5. After about 6 hours the pH was stable and the product was filtered, washed with (2 10 ml) of ethanol-water (3:7, v:v) and then dried in a vacuum oven at room temperature for 24 hours, 42.97 g (92% yield), m.p. 50—52C.

### Example 2

The procedure of Example 1 was repeated, the pH being maintained as indicated to give the desired product at the indicated yield.

| pH | yield % |
|---|---|
| 6.5 | 88.6 |
| 7.0 | 89.3 |
| 7.5 | 92.6 |
| 8.0 | 88.0 |

Example 3

The procedure of Example 1 was repeated with exception that the required acid (17.8 ml) was added all at once 30 minutes after the completion of the ethyl chloroformate addition was complete. No crystalline product was formed, only a clear orange oil formed.

## Claims

1. A process for preparing bis(ethoxythiocarbonyl)sulfide which comprises contacting an aqueous-ethanolic solution of sodium ethyl xanthate with ethyl chloroformate such that the molar ratio of sodium ethyl xanthate to ethyl chloroformate is about 2.1—2.3 to 1 and maintaining the pH of the mixture at about 7.5 by the addition of acid until the reaction is substantially complete.

2. The process of claim 1, where the volume ratio of ethanol to water is from about 2—3 to about 3—2.

3. The process of claim 2, wherein the pH is maintained with 12*N* hydrochloric acid.

## Patentansprüche

1. Verfahren zum Herstellen von Bis(äthoxythiocarbonyl)sulfid, das das Inberührungbringen einer wässerig-äthanolischen Lösung von Natriumäthylxanthat mit Äthylchlorformiat derart, daß das Molverhältnis von Natriumäthylxanthat zu Äthylchlorformiat etwa 2,1 bis 2,3 zu 1 beträgt, und das Halten des pH der Mischung durch Zusatz von Säure bei etwa 7,5 bis die Reaktion im wesentlichen beendet ist, umfaßt.

2. Verfahren nach Anspruch 1, worin das Volumsverhältnis von Äthanol zu Wasser etwa 2 bis 3 zu etwa 3 bis 2 beträgt.

3. Verfahren nach Anspruch 2, worin der pH mit 12n Salzsäure aufrechterhalten wird.

## Revendications

1. Procédé de préparation de sulfure de bis(éthoxythiocarbonyle), qui consiste à mettre en contact une solution aqueuse-éthanolique d'éthylxanthate de sodium avec le chloroformiate d'éthyle de sorte que le rapport molaire de l'éthylxanthate de sodium au chloroformiate d'éthyle soit d'environ 2,1—2,3 à 1, et à maintenir le pH du mélange à environ 7,5 par addition d'un acide jusqu'à ce que la réaction soit pratiquement totale.

2. Procédé suivant la revendication 1, dans lequel le rapport en volume de l'éthanol à l'eau est d'environ 2—3 à environ 3—2.

3. Procédé suivant la revendication 2, dans lequel le pH est maintenu avec de l'acide chlorhydrique 12*N*.